# EUROPEAN PATENT APPLICATION

(11) **EP 1 384 708 A1**
(43) Date of publication of application: **28.01.2004**
(21) Application number: 02016726.8
(22) Date of filing: 26.07.2002
(51) Int. Cl.: C07C 213/10, C07C 213/00, C07C 215/64, A61K 31/22, A61P 9/12

(54) **Process for the manufacture of form I of nolomirole hydrochloride**

(71) Applicant: CHIESI FARMACEUTICI S.p.A., I-43100 Parma (IT)
(72) Inventor: Pighi, Roberto, 43100 Parma (IT); Pivetti, Fausto, 43100 Parma (IT)
(74) Representative: Banfi, Paolo

(57) **Abstract**

A process for the preparation and isolation of the pharmaceutical chemical compound nolomirole hydrochloride in a highly purified crystalline Form I, as well as pharmaceutical preparations thereof for the treatment of patients suffering of cardiovascular diseases such as congestive heart failure and hypertension are described.

## Description

The present invention relates to the pharmaceutical chemical compound nolomirole hydrochloride in a highly purified crystalline Form I, to the process for its preparation and isolation as well as to pharmaceutical preparations thereof for the treatment of patients suffering of cardiovascular diseases such as congestive heart failure and hypertension.

In particular the present invention relates to a crystalline composition having a content of nolomirole hydrochloride Form I of at least 97% by weight of the active ingredient, preferably higher or equal than 98% and at least 90% w/w polymorphic purity, to the process for its preparation and isolation as well as to pharmaceutical preparations thereof for the treatment of patients suffering of cardiovascular diseases such as congestive heart failure.

(±)-5,6-diisobutyroyloxy-2-methylamino-1,2,3,4-tetrahydronaphthalene hydrochloride, (hereinafter indicated with its approved INN nolomirole hydrochloride) is a DA₂ dopaminergic receptor/α₂-adrenoceptor agonist, which has been claimed to be useful for the treatment of cardiovascular diseases (WO 96/29065) and in particular for the treatment of congestive heart failure.

Nolomirole is conveniently formulated in tablets for oral administration. The hydrochloride salt is particularly suitable for the preparation of tablets.

The daily dosage is comprised between 2.5 and 20 mg.

Nolomirole is a pro-drug which, after oral administration, is rapidly and completely converted, during absorption, by the esterases contained in the intestinal mucosa into its desesterified form, namely (±)-5,6-dihydroxy-2-methylaminotetralin, indicated hereinafter with the experimental code CHF 1024.

CHF 1024 also turned out to be one of the main occurring impurities and degradation products. Although the CHF 1024 is the active moiety, it is nevertheless important for pharmaceutical and therapeutic purposes that the active ingredient is present as pro-drug (nolomirole) with the highest as possible chemical purity.

Nolomirole has been first described as hydrobromide salt in GB 2,123,410 among a series of aminotetralin derivatives (tetralin is another name for 1,2,3,4-tetrahydro-naphthalene) disclosed as potential antibronchospastic agents. In the description, an indication for the preparation of the hydrobromide on a laboratory scale is provided in analogy to the pivaloyl derivative. Said procedure envisions precipitation from a mixture of ethyl ether and petroleum ether. The product has been characterised in terms of NMR spectrum in solution and melting point. The solvents used for its preparation cannot be utilised on an industrial scale; moreover, although it is reported that the product is obtained as a white crystalline powder, once moving on a bigger scale, a significant percentage of amorphous is obtained.

EP 788472 in the name of the applicant and EP 534536 in the name of Zambon refer, respectively, to processes for the preparation of 5,6 dihydroxy-2-amino-tetralins and N-alkyl derivatives thereof and 5,6-dimethoxy-2-amino-tetralin as intermediates useful for obtaining pharmacologically active chemical compounds. In none of them processes for the preparation and isolation of acyl derivatives such as nolomirole are mentioned.

In a study presented as a poster at the XX^{th} National Congress of AICAT (Rome, December 14-18, 1998), mainly aimed at investigating the thermal behaviour of nolomirole hydrochloride racemate (quoted with its experimental code CHF 1035) and its enantiomers, preliminary results about its polymorphism were disclosed. In the poster it is stated that Form I can be obtained from acetone and ethanol after fast cooling, Form II from acetone after slow cooling and from III from methyl-ethyl-ketone. No details about the crystallisation conditions are reported nor on the process of preparation of nolomirole hydrochloride.

In Giordano et al (*J Pharm Sci* 2001, 90(8), 1154-1163), the polymorphism of nolomirole hydrochloride has been thoroughly investigated. Thermal, spectroscopic, and X-ray diffraction analysis confirms that three differing crystal forms by recrystallization procedures from common organic solvents can be obtained.

Recrystallizations have been performed on a laboratory scale according to the following procedures: 10 ml of the solvent under examination were saturated with the product, holding the temperature slightly below the boiling point. The hot suspension was then rapidly filtered into a flask maintained at room temperature (RT) or ice-cooled (IC). In all cases the filtrate was allowed to reach spontaneously RT and to equilibrate overnight.

From ethanol and methanol as well as from methylethylketone, a single solid phase has been obtained whatever recrystallisation procedure is used; on the contrary, from acetone two solid phases were obtained depending on the suspension was maintained at RT or IC. According to Giordano, Form I can be obtained from ethanol and methanol or acetone at room temperature, Form II from methylethylketone and Form III from acetone ice cooling.

On the contrary, we have found that:
- Form I cannot be prepared from ethanol/methanol in a highly chemical pure form since such solvents can give rise to trans-esterification reactions and thus to significant amounts of the de-esterified degradation products; it can be prepared from acetone by ice cooling;
- Form II can be obtained from acetone at RT;
- Form III is obtained from methylethylketone.

Moreover it has been found that Form II and Form III have not the desirable characteristics with respect both of their manufacturing process and their pharmaceutical utilisation since they are obtained as extremely fine crystals associated with partially amorphous particles. During isolation, fine crystals are indeed hardly filtered off to detriment of the product yield; moreover, due to this characteristic, said forms strongly retain the solvent(s) of crystallisation so they cannot easily be dried. The disadvantages of having products at least partially amorphous have already been discussed above. Form III is also a metastable form as it undergoes a transformation into Form I within 24 hours either in acetone or methylethylketone.

In view of these findings, it appears that the only form of nolomirole hydrochloride suitable for pharmaceutical development is Form I. Accordingly, there is a need of a process for preparing a nolomirole hydrochloride able to give rise to the Form I with high polymorphic purity in order to fulfil the requirements described above.

There is thus a need of a nolomirole hydrochloride in as chemically pure and as highly crystalline condition as possible in order to fulfil pharmaceutical requirements and of a process for its preparation.

In general terms, chemical purity is a very important issue for pharmaceutical products.

In the case of the product of the invention, the main occurring impurities are the monoester 5(6)-isobutyroyloxy-2-methylamino-1,2,3,4-tetrahydro-naphthalene (CHF 2101) and the starting material, i.e. the free cathecol derivative (CHF 1024) which, during storage, is easily oxidized to quinonic derivatives as evidenced by the slight coloring of the product.

In general terms, in a pharmaceutical product impurities should be kept as lowest as possible as they could be detrimental to the potency of the active ingredient and they could also be responsible of unwanted side effects.

In this particular case the presence of oxidative products gives also rise to a colouring of the raw material.

High crystallinity is another important issue as amorphous or prevalently amorphous compounds are not suitable for pharmaceutical use due to the fact that potential crystallisation during handling and storage is always present. Such crystallization, in turn, can be responsible for phenomena such as post- compression hardening of tablets (Elamin et al Int J Pharm 1994, 108, 729-752). Moreover amorphous or partially amorphous materials absorb water in larger amounts than crystalline ones (Hancock et al. J. Pharm. Sci. 1997, 86, 1-12); so formulations containing active ingredients such as nolomirole hydrochloride, whose chemical stability is particularly sensitive to the humidity content due to the presence of ester bonds which can be subjected to hydrolysis reactions, could face stability problems during long term storage.

Polymorphic purity is a further important issue since different polymorphs have different physical properties which in turn can affect both stability and bioavailability. Among others, it has been reported that different physical forms (e.g. polymorphs) can have different hydrolysis and rate profiles (Waterman K et al *Pharm Dev Tech* 2002, 7, 113-146) so that the stability of molecules such as nolomirole hydrochloride could be dramatically affected by the polymorphic purity.

The process for the preparation of nolomirole hydrochloride also needs to be one suitable for operating on an industrial scale and that the solvent(s) for crystallization should be readily and almost completely removable. Moreover, the product should be in a form that is readily filtered off and easily dried. Finally, since the content of nolomirole hydrochloride per unitary composition is relatively low (2.5-5 mg), it would be highly advantageous to obtain a product with a particle size less than 300 m, preferably between 100 and 300 m. Said particle size is optimal for having either good flowability characteristics and an homogenous distribution of the active ingredient in the tablets.

### OBJECT OF THE INVENTION

It is a first object of the invention to provide nolomirole hydrochloride of at least 95% chemical purity. It is a further object of the invention to provide nolomirole hydrochloride Form I of at least 97% chemical purity, preferably of at least 98%. In a preferred embodiment of the invention it is provided nolomirole hydrochloride Form I of at least 97% chemical purity, preferably of at least 98% containing an amount equal or less than 0.3% by weight of 5,6-dihydroxy-2-methylamino-1,2,3,4-tetrahydro-naphthalene hydrochloride (CHF 1024) and/or a content of 5(6)-isobutyroyloxy-2-methylamino-1,2,3,4-tetrahydro-naphthalene (CHF 2101) which is less or equal than 1.0% by weight.

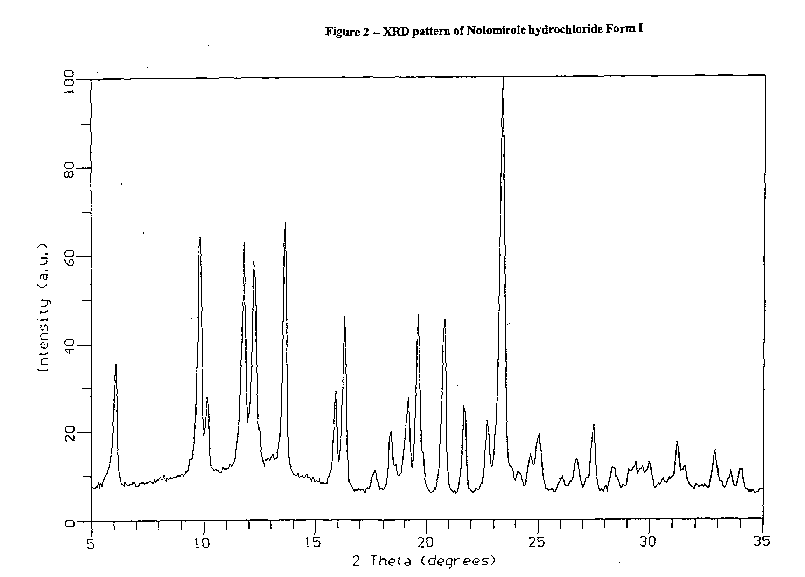

Form I and Form II according to the present invention are identified from the Raman spectra and the powder X-ray diffraction pattern reported in Figures 2,3,4,5 respectively.

Another object of the present invention is to provide a manufacturing process, which can be applied on an industrial scale for preparing nolomirole hydrochloride of at least 95% chemical purity. A further object of the invention is a purification process of nolomirole hydrochloride to obtain the Form I characterized by high crystallinity as well as high chemical and polymorphic purity, by at the same time fulfilling the other desirable features described above.

According to a first embodiment there is provided a process which comprises the steps of:
i) reacting (±) 5,6-dihydroxy-2-methylamino- 1,2,3,4-tetrahydro-naphthalene hydrochloride (CHF 1024) with isobutyroyl chloride optionally in the presence of a suitable solvent to yield raw nolomirole;
ii) removing any volatile substance from the raw reaction product and dissolving the oily residue with an organic solvent;
iii) precipitating nolomirole as hydrochloride salt from the solution by adding hydrochloric acid;
wherein the organic solvent of step ii) is an ether solvent, preferably isopropyl ether or mixtures thereof with non-alcoholic solvents and step iii) is carried out by bubbling gaseous hydrochloric acid to avoid the use of water which might favour the hydrolysis of the ester bonds.

According to a second embodiment there is provided a process for the (re)-crystallization of nolomirole hydrochloride by dissolving the product in a non alcoholic solvent with a boiling point lower than 70°C, preferably lower than 60°C, then cooling the solution to a temperature lower than 20°C, preferably equal or lower than 5°C at a speed higher than 1°C/min.

As said before, the synthesis of 5,6-dihydroxy-2-methylamino-1,2,3,4-tetrahydro-naphthalene hydrochloride (CHF 1024) is described in EP 788472.

The synthesis of nolomirole hydrochloride is effected by the conversion of CHF 1024 hydrochloride into the final product by reaction with isobutyroyl chloride, using a suitable solvent. The elimination of the solvent excess from the reaction raw product leads to the formation of an oily mass. As reported above, the process for the preparation of nolomirole hydrochloride needs to be suitable for operating on an industrial scale. In particular it would be desirable that the hydrochloride should be prepared with gaseous hydrochloric acid.

It has been found that nolomirole could be precipitated as hydrochloride from its oily mass by addition of gaseous hydrochloric acid in the presence of ether solvents. It has also been even more surprisingly found that, among them, isopropyl ether is the most suitable since in the presence of another widely used ether solvent, i.e methyl-*tert*-butyl ether, nolomirole might experience a significant degradation to monoester (CHF 2101) and free cathecol (CHF 1024) derivatives. We have indeed found that methanol can form due to the degradation of methyl *tert*-butyl ether in acidic environment; without being limited by the theory, we have hypothesized that the formed methanol reacts with nolomirole by parasitic trans-esterification giving rise to the two aforementioned degradation products *(vide supra).*

Although raw nolomirole hydrochloride is further subjected to a purification step by crystallization, nevertheless the amount of the above mentioned impurities should be maintained as low as possible since they are difficult to be completely eliminated from the finished product due to their close structural similarity.

It has also been found that a highly chemical and polymorphic purified, crystalline composition of nolomirole hydrochloride Form I can only be obtained using a non alcoholic solvent with a boiling point lower than 70°C, preferably lower than 60°C and after cooling the solution of raw nolomirole to a temperature lower than 20°C, preferably equal or lower than 5°C at a speed higher than 1°C/min.

The preferred non alcoholic solvent is acetone. Contrary to what reported in Giordano et al, when the crystallization of nolomirole hydrochloride from acetone is carried out at room temperature (higher than 20°C), it predominantly gives rise to the form II, which is unsuitable for pharmaceutical utilization.

Moreover, although nolomirole hydrochloride as polymorphic pure Form I can be obtained from ethanol and methanol as well, such solvents, as reported above, are not suitable as they give rise to trans-esterification reactions and thus to significant amounts of the de-esterified degradation products mentioned above, namely CHF 2101 and CHF 1024.

The (re)-crystallization process of the invention, instead, allows to obtain predominantly polymorph I of nolomirole hydrochloride (polymorphic purity higher than 90%) in a highly crystalline form with a very high chemical purity (higher than 97%).

Advantageously, it also gives rise to particles with an inherent particle size optimal for nolomirole hydrochloride to be conveniently formulated in tablets for oral administration. The particles of the active ingredient are uniformly incorporated in the mixture before tabletting by simple mixing so avoiding any further treatment such as milling which may disturb their crystallinity.

The present invention also provides a process for the conversion of Form II of nolomirole hydrochloride to Form I.

The process can be carried out using similar conditions of (re)crystallisation as those described above which give purified Form I. According to this way, Form II is dissolved in a non alcoholic solvent having a boiling point lower than 70°C, preferably lower than 60°C, such as acetone, by warming, followed by cooling at a temperature lower than 20°C, preferably lower than 15°C till complete crystallisation. In order to speed up the process, the solution may be seeded with few crystals of Form I prior to the initiation of product crystallisation. Alternatively, the process can be carried out by: i) suspending Form II in a non alcoholic solvent having a boiling point lower than 70 °C, preferably lower than 60°C, such as acetone, in the presence of at least few crystal of Form I; ii) keeping the suspension under stirring at a temperature lower than 20°C, preferably 10°C for at least one hour, preferably more than two hours, more preferably for eight hours; filtering the resulting product.

Another object of the present invention refers to the use of nolomirole hydrochloride Form I of at least 97% chemical purity for the preparation of pharmaceutical compositions for the treatment of patients suffering form cardiovascular diseases such as hypertension and congestive hearth failure.

A further object of the present invention are pharmaceutical compositions, preferably tablets for oral administration, further comprising pharmaceutically acceptable excipients, advantageously containing between 2.5 mg and 10 mg of the active ingredient per unit dose, preferably between 2.5 mg and 5 mg.

### DETAILED DESCRIPTION OF THE INVENTION

The characteristics of the process of the invention for preparing nolomirole hydrochloride and nolomirole hydrochloride Form I and the features of the product obtained will be more apparent from the following detailed description.

Generally, the last step during the synthesis of nolomirole hydrochloride is the conversion of 5,6-dihydroxy-2-methylamino-1,2,3,4-tetrahydro-naphthalene hydrochloride into the product by reaction with isobutyryl chloride, using a suitable solvent, followed by the elimination of the solvent excess from the reaction raw product till an oily mixture is obtained. The term suitable solvent means any volatile polar acidic solvent, preferably halogenated acetic acid. The preferred one is trifluoroacetic acid.

Advantageously, nolomirole is precipitated as hydrochloride from the oily mixture by bubbling gaseous hydrochloric acid in the presence of an ether solvent. Preferably, gaseous hydrochloric acid is bubbled for at least 15 minutes, more preferably for 60 minutes keeping the temperature under 20°C, more preferably at 5°C.

As an ether solvent, isopropyl ether, ethyl ether, dioxane, tetrahydrofurane, 2-methoxyethyl ether and 2-ethoxyethyl ether can be advantageously used. Preferably, pure isopropyl ether or mixtures thereof with other non alcoholic solvents such as acetone and toluene, preferably in a ratio comprised between 10:1 to 3:1 v/v are used. More preferably, a mixture of isopropyl ether: acetone in a ratio 5:1 is used. Although is not preferred, methyl tert-butyl ether can also be utilized.

Advantageously the process of (re)-crystallization for the preparation of nolomirole hydrochloride Form I starting from either raw nolomirole hydrocholoride or nolomirole hydrocholoride Form II is carried out as follows: i) dissolving in a suitable flask the product in a non alcoholic solvent having a boiling point lower than 70°C, preferably lower than 60°C such as acetone at a temperature from about 30°C to reflux temperature, preferably at 50°C; ii) cooling the solution, preferably under stirring, by standard methods and/or by concentration under vacuum to a temperature lower than 20°C, preferably lower than 15°C, even more preferably lower or equal to 5°C at a speed higher than 1°C/min, preferably equal or higher than 2°C/min. Although they are not advisable for environmental reasons, halogenated solvents such as chloroform could be theoretically used.

The amount of solvent is at least 3 part by volume per part of product. Higher amounts of solvents and generally up to 10 parts by volume can be used. Amounts higher than 10 volumes are not useful from an economical point of view because large size apparatus would be necessary. The most preferred solvent is acetone.

The flask will be advantageously maintained at 5°C and the precipitation will typically be accomplished within 5 hours. However the time required will vary depending on such factors such as total volume of solution, size of batch and presence or absence of stirring. Methods known in the art may be used to enhance any aspect of the process. For example, as reported above for crystallization of Form I starting from Form II, the solution may be seeded with few crystals of Form I prior to the initiation of product crystallisation.

Generally, nolomirole hydrochloride Form I can be collected by any standard method known in the art such as by filtration, filtration under vacuum, or decantation and drying.

Normally, nolomirole hydrochloride Form I obtained by the process of the invention has a chemical purity, as determined by HPLC of at least 97% by weight of the active ingredient, preferably higher or equal than 98%, with a content of CHF 1024 which is less or equal than 0.3% by weight and/or a content of CHF 2101 which is less or equal than 1.0% by weight.

Advantageously, the polymorphic purity and the degree of crystallinity are both higher than 90% by weight, preferably higher than 95% as determined by powder X-ray diffraction. After isolation of the finished product, advantageously, at least 95% of the particles has a diameter equal or lower than 300 µm, preferably comprised between 100 and 300 m, as determined by laser diffraction (Malvern).

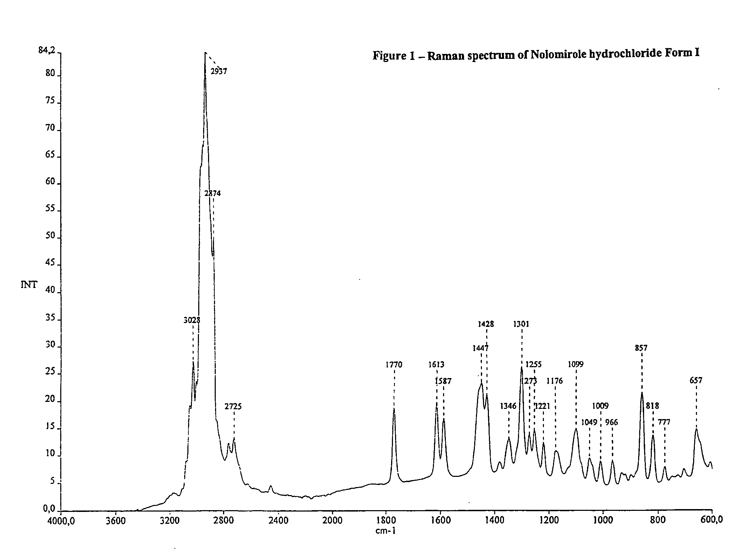

Form I of nolomirole hydrochloride produced according to the process of the invention can be characterised by its Raman spectrum and its X-ray powder diffraction (XRD) pattern (Figs 1 and 2). The FT-Raman spectrum, obtained by simply packing the powder into a cup, shows the following main peaks in the 3500-700 cm⁻¹ range (accuracy ± 1 cm⁻¹):
2937 cm⁻¹ (vs); 1771 (s); 1614 (s); 1588 (s); 1446 (s); 1426 (s); 1302 (s);1274 (m); 1225 (m); 1221 (m); 1098 (br, m); 1009 (w); 966 (w); 856 (s); 818 (m)
Legend: vs = very strong; s = strong; m = medium; w = weak; br = broad

The interplanar distances and relative peak intensities (in brackets) of the powder XRD pattern are reported as follows (depending on the packing conditions, the relative intensities can change):
14.35 d/Å (64); 8.93 (18); 8.63 (6); 7.46 (34); 7.19 (100); 7.13 (5); 7.04 (3); 6.46 (19); 5.55 (6); 5.40 (12); 4.82 (4); 4.66 (3); 4.62 (7); 4.52 (11); 4.27 (18); 4.07 (5); 3.90 (3); 3.80 (28); 3.60 (6); 3.55 (3); 3.24 (4); 2.86 (3).

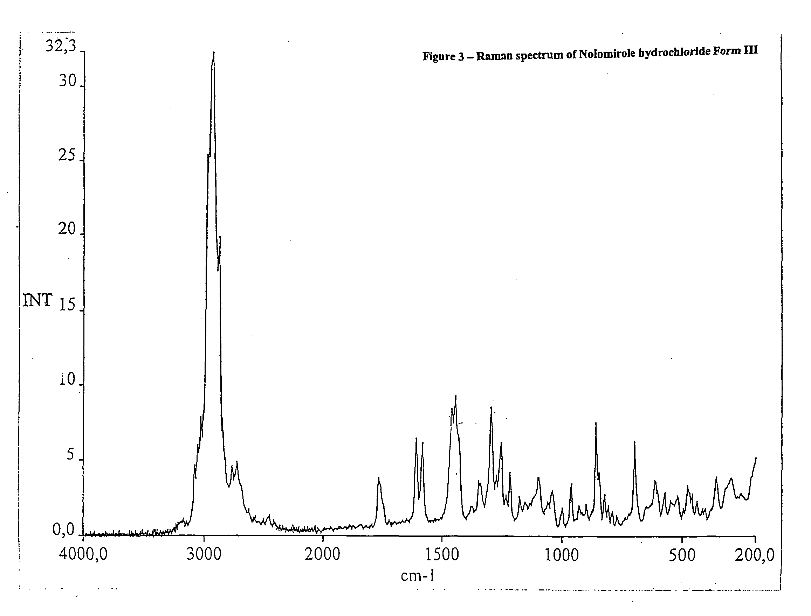

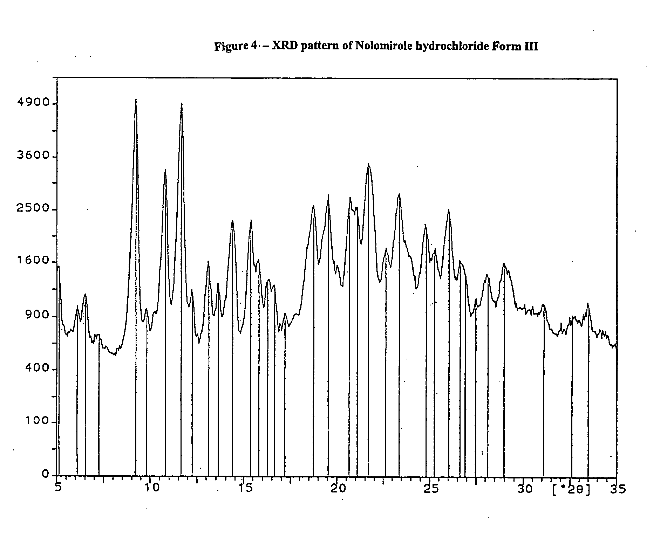

For comparison Figures 3 and 4 show the Raman and XRD fingerprints of Form II.

The following examples better illustrate the invention.

### EXAMPLES

Example 1 - Process for obtaining (±)-5,6-diisobutyroyloxy-2-methylamino-tetralin hydrochloride (nolomirole hydrochloride) starting from (±) 5,6-dihydroxy-2-methylamino-1,2,3,4-tetrahydro-naphthalene (CHF 1024) hydrochloride.

Slowly add, in 2 hours, a molar excess of isobutyryl chloride to a suspension of CHF 1024 in trifluoroacetic acid, always keeping temperature at 25±2°C.

Then heat the solution to 85 ± 2°C for 1 hour, thus obtaining a full dissolution.

Cool to 50 ± 5°C, distil the maximum part of trifluoracetic acid under vacuum and add toluene to the oily residue: then, distil again to oil.

Repeat three times both addition and distillation of toluene, then dissolve the oily residue in a mixture of isopropyl ether: acetone 5:1 v/v. Let gaseous hydrochloric acid bubbling for 60 minutes keeping temperature under 20°C.

Raw nolomirole hydrochloride precipitates as a white crystalline solid and the suspension is kept at 10±2°C for three hours, then at 5±3°C for further five hours and finally centrifuged. The obtained raw nolomirole hydrochloride is dried under vacuum at 60 ± 2°C.

The purity of the compound obtained is about 95% both upon ¹H NMR and HPLC analysis.

¹H NMR (CH₃OH-d4, ppm): 1.28 (d, 6H); 1.35 (2 x d, 6H); 1.82 (m, 1H), 2.30 (m, 1H); 2.6-3.0 (m, 5H); 2.80 (s, 3H); 3.30 (dd, 1H); 3.48 (m, 1H), 7.0-7.2 (AB syst, 2H).

¹³C NMR (CH₃OH-d4, ppm): 19.3; 19.4; 22.9; 25.6; 30.9; 32.2; 35.0; 55.9; 122.5; 128.2; 130.5; 132.4; 141.6; 142.5; 175.5; 176.6.

Legend: d = doublet; dd= doublets of doublet; m = mutiplet; s = single

### Example 2 - Process for obtaining nolomirole hydrochloride Form I starting from raw nolomirole hydrochloride

Raw nolomirole hydrochloride is purified by crystallisation on acetone. The solid substance is dissolved in 10 volumes of solvent at 50°C, filtered, then the limpid solution is cooled to a temperature of 5°C upon concentration under vacuum at a speed of 2°C/min. The temperature is kept for five hours at 5 ± 2°C, then centrifuged. The solid is dried under vacuum, at 60 ± 2°C.

Assay = 99.1% (HPLC); melting range = 199-202°C (DSC).

## Claims

1. A process for preparing (±)-5,6-diisobutyroyloxy-2-methylamino-tetralin (nolomirole) hydrochloride which comprises the steps of:
**i)** reacting (±) 5,6-dihydroxy-2-methylamino-1,2,3,4-tetrahydronaphthalene hydrochloride (CHF 1024) with isobutyroyl chloride optionally in the presence of a suitable solvent to yield raw nolomirole;
ii) removing any volatile substance from the raw reaction product and dissolving the oily residue with an organic solvent;
iii) precipitating nolomirole as hydrochloride salt from the solution by adding hydrochloric acid;
wherein the organic solvent of step ii) is an ether solvent, preferably isopropyl ether or mixtures thereof with non-alcoholic solvents and step iii) is carried out by bubbling gaseous hydrochloric acid to avoid the use of water which might favour the hydrolysis of the ester bonds.

2. A process according to claim 1 wherein the step i) is carried out in the presence of a volatile polar acidic solvent such as trifluoracetic acid.

3. A process according to claims 1-2 wherein the ether solvent is selected from isopropyl ether, ethyl ether, dioxane, tetrahydrofurane, 2-methoxyethyl ether and 2-ethoxyethyl ether.

4. A process according to claim 3 wherein the ether solvent is isopropyl ether or mixtures thereof with non-alcoholic solvents such acetone or toluene.

5. A process according to any of preceding claims wherein nolomirole hydrochloride is of at least 95% chemical purity.

6. A process for preparing crystalline nolomirole hydrochloride Form I by dissolving nolomirole hydrochloride in a non alcoholic solvent with a boiling point lower than 70°C then cooling the solution to a temperature lower than 20°C at a speed higher than 10C/min.

7. A process according to claim 6 wherein the non alcoholic solvent is acetone and the product is dissolved in said solvent at a temperature from about 30°C to reflux temperature.

8. A process according to claims 6-7 wherein the solution is cooled at a temperature lower than 15°C.

9. A process according to claims 7-8 wherein the solution is cooled at a temperature lower than 5°C at a speed equal or higher than 2°C/min.

10. A process for preparing a crystalline nolomirole hydrochloride Form I which comprises the step of dissolving nolomirole hydrochloride Form II in a non alcoholic solvent with a boiling point lower than 70°C then cooling the solution at a temperature lower than 20°C.

11. A process for preparing a crystalline nolomirole hydrochloride Form I which comprises the steps of: i) suspending nolomirole hydrochloride Form II in a non alcoholic solvent having a boiling point lower than 70°C in the presence of at least few crystal of Form I; ii) keeping the suspension under stirring at a temperature lower than 20°C, preferably 10°C for at least one hour, preferably more than two hours, more preferably eight hours; filtering the resulting product.

12. A process according to claims 10-11 wherein the non alcoholic solvent is acetone.

13. A process according to claims 6-12 for preparing crystalline nolomirole hydrochloride Form I of at least 97% by weight chemical purity.

14. A process according to claim 13 wherein the amount of 5,6-dihydroxy-2-methylamino-1,2,3,4-tetrahydro-naphthalene hydrochloride is equal or less than 0.3% by weight and/or the amount of 5(6)-isobutyroyloxy-2-methylamino- 1,2,3,4-tetrahydro-naphthalene is less or equal than 1.0% by weight.

15. A process according to claims 6-14 wherein the polymorphic purity and the crystallinity are higher than 90% by weight.

16. Crystalline nolomirole hydrochloride Form I of at least 97% by weight chemical purity.

17. Crystalline nolomirole according to claim 16 wherein the amount of 5,6-dihydroxy-2-methylamino- 1,2,3,4-tetrahydro-naphthalene hydrochloride is equal or less than 0.3% by weight and/or the amount of 5(6)-isobutyrroyloxy- 2-methylamino-1,2,3,4-tetrahydro-naphthalene is less or equal than 1.0% by weight.

18. Crystalline nolomirole according to claims 16 and 17 wherein the polymorphic purity and the crystallinity are higher than 90% by weight.

19. Nolomirole hydrochloride of at least 95% by weight chemical purity as an intermediate for preparing crystalline nolomirole hydrochloride Form I

20. A pharmaceutical composition comprising nolomirole hydrochloride of claims 16-18 as active ingredient in combination with at least one pharmaceutical acceptable excipient.

21. A pharmaceutical composition according to claim 20 in the form of tablet for oral administration containing between 2.5 and 5 mg of nolomirole hydrochloride per unit dose.

22. Use of nolomirole hydrochloride of claims 16-18 for the preparation of pharmaceutical compositions for the treatment of patients suffering from cardiovascular diseases such as hypertension and congestive heart failure.
